# EUROPEAN PATENT SPECIFICATION

(11) **EP 3 108 873 B1**
(45) Date of publication and mention of the grant of the patent: **21.06.2017**
(21) Application number: 14885101.7
(22) Date of filing: 04.12.2014
(51) Int. Cl.: A61K 8/98, A61Q 19/02, A61K 8/64, A61K 8/19

(54) **METHOD FOR SEPARATING AND PREPARING PEARL EXTRACT FROM PEARLS**
VERFAHREN ZUR TRENNUNG UND HERSTELLUNG VON PERLENEXTRAKTEN AUS PERLEN
PROCÉDÉ DE SÉPARATION ET DE PRÉPARATION D'EXTRAIT DE PERLES À PARTIR DE PERLES

(30) Priority: 14.03.2014 CN 201410097089
(43) Date of publication of application: 28.12.2016
(73) Proprietor: Osmun Biological Co., Ltd., Mogan Shan High-tech Zone Huzhou Zhejiang 313200 (CN)
(72) Inventor: YANG, Anquan, Huzhou Zhejiang 313200 (CN); WANG, Jing, Huzhou Zhejiang 313200 (CN); ZHANG, Lihua, Huzhou Zhejiang 313200 (CN); LUO, Jianfen, Huzhou Zhejiang 313200 (CN); XIE, Min, Huzhou Zhejiang 313200 (CN)
(74) Representative: Sun, Yiming
(86) International application number: PCT/CN2014/093042
(87) International publication number: WO 2015/135346

(56) References cited:
- CN-A- 1 152 439
- CN-A- 1 152 439
- CN-A- 103 333 222
- DATABASE WPI Week 201419 Thomson Scientific, London, GB; AN 2013-W87810 XP002763153, & CN 103 333 222 A (ZHEJIANG PHOENIX BIRD PEARL BIOLOGICAL) 2 October 2013 (2013-10-02)
- DATABASE WPI Week 201146 Thomson Scientific, London, GB; AN 2011-G01712 XP002763154, & CN 102 018 727 A (GUILIN LAYN NATURAL INGREDIENTS CORP) 20 April 2011 (2011-04-20)
- DATABASE WPI Week 200837 Thomson Scientific, London, GB; AN 2008-F55640 XP002763155, & CN 101 100 689 A (HAINAN JINGRUN PEARL BIOLOGICAL TECHNOLO) 9 January 2008 (2008-01-09)
- DATABASE WPI Week 200411 Thomson Scientific, London, GB; AN 2004-099612 XP002763156, & CN 1 425 388 A (OUSHIMAN IND CO LTD ZHEJIANG) 25 June 2003 (2003-06-25)
- DATABASE WPI Week 201419 Thomson Scientific, London, GB; AN 2014-D29407 XP002763157, & CN 103 446 185 A (BEIHAI YUANLONG PEARL CO LTD) 18 December 2013 (2013-12-18)
- DATABASE WPI Week 201046 Thomson Scientific, London, GB; AN 2010-H35951 XP002763158, & CN 101 720 917 A (WU W) 9 June 2010 (2010-06-09)
- DATABASE WPI Week 200919 Thomson Scientific, London, GB; AN 2009-F26547 XP002763159, & CN 101 357 141 A (HUNAN JINZHU BIOTECHNOLOGY CO LTD) 4 February 2009 (2009-02-04)
- DATABASE WPI Week 199530 Thomson Scientific, London, GB; AN 1995-224721 XP002763160, & CN 1 086 713 A (BAOQING NUTRITIOUS PREPARATION FACTORY) 18 May 1994 (1994-05-18)

## Description

### Technical field

This invention relates to a pearl processing method, particularly a method to separate and prepare pearl abstract from pearls.

### Background technology

At present, in the application of pearl powder, there are many a problems including potential safety hazard related to particle size of the powder, poor mobility, insolubility in water and difficulty of release of functional components. Those pose directly impact on the application field of pearl powder. To solve the problems, the water-soluble pearl powder and hydrolyzed pearl solution are becoming a trend. However, in this process, we simply compound the original components or hydrolyze the original components in an extensive way, and we haven't studies the functions of each component, and seldom carry out fine separation and purification.

A Chinese invention patent (application No.: 93117551.8; date of application: September 18, 1993) reveals a method in which the sodium hydroxide is used to neutralize, filter, concentrate, crystallize, dry and smash the pearl and pearl layer powder as the raw materials through organic acid reaction, to prepare pearl calcium and the pearl calcium obtained. The product contains calcium of about 20%wt, and various amino acids, which are soluble in water, and easily absorbed by human body. Therefore, it is an ideal source of calcium for human body.

A Chinese invention patent (application No.: 200710035508.5; date of application: August 3, 2007) reveals a kind of water-soluble mixed active pearl calcium and its preparation method. The steps are as follows: ① Smash and grind the pearl and swan mussel and mix them up; ② Add water and gum and grind them into slurry mixture; ③ Add bio-enzyme for enzymolysis; ④ Carry out negative-pressure filtration to obtain filter liquor; ⑤ Place the filter residue into an acidolysis tank and add a proper amount of lactic acid; heat it up and stir for 0.5 to 1 hour; ⑥ Obtain filter liquor through negative pressure filtration; ⑦ Place the filter residue obtained in Step ⑥ into the acidolysis tank and add a proper amount of food phosphates; stir it up and hydrolyze and filter it to obtain hydrolysate 3; ⑧ Combine filter liquor 1, filter liquor 2 and hydrolysate 3, and stir them up and regulate pH value to 5 to 6 and then the water-soluble mixed pearl calcium liquid is obtained; ⑨ Concentrate and dry the calcium liquid obtained in Step ⑧ and smash it into powder, and then the water-soluble mixed active pearl calcium powder is obtained.

A Chinese invention patent (application No.: 200910186567.1; date of application: November 27, 2009) reveals a method used to prepare a kind of high-quality water-soluble pearl calcium powder and its application products. The preparing method of the water-soluble pearl calcium powder includes: Select coarse powder made from the pearl mussel and make it into filter cakes. The ratio of pearl to pearl layer powder (weight) should be 0.05-2.95:2.95-0.05; add potable water and stir them up and grind them. Add some portable water to wash the grinding body and mix them with sizing agent. Add it into the enzymolysis tank. Add food-grade neutral protease of bacillus subtilis after heating for enzymolysis. The filter liquor is the composite amino acids (Liquid A). Place the filter cake into the enzymolysis tank and add some lactic acid. Heat up the tank and stir it for hydrolysis. After the solid content is basically dissolved, stop stirring, and regulate pH value to 6.3 to 6.6. After filtering, the filter liquor will be the water-soluble pearl calcium liquid (Liquid B). Remove the crystal water at temperature of 120°C to obtain anhydride. Spray dry Liquid A and Liquid B to obtain water-soluble pearl powder.

A Chinese invention patent (application No.: 201310383763.4; date of application: August 29, 2013) reveals a method used to prepare soluble pearl calcium from pearl shell. Firstly, the pearl shell is made into suspension; prepare solution using dietary alkali and taurine. Heat the dietary alkali solution to 55 to 85°C, with the volume ratio of suspension to dietary alkali solution being 1:3-6. Add the suspension into the dietary alkali solution and mix them up. Keep the temperature at 55°C to 85°C for 60 to 72 hours. The volume ratio of suspension to taurine solution is 1:3-6. Then add taurine solution and mix them up until the pearl shell fine powder is completely dissolved to obtain the calcified liquid, which will be concentrated and dried to obtain the product.

A Chinese invention patent (application No.: 01139157.X; date of application: December 19, 2001) reveals a method to prepare pearl powder by biological enzymolysis, which features use of lactic water solution as the solvent to solve the pearl and regulate the pH value of the solution to near-neutrality and then back to weak acidity with acid. Afterwards, a proper amount of biological active enzyme to catalytic-decompose its protein to obtain the enzymolysis pearl glue solution. Then spraying dry is adopted to quickly dry the enzymolysis pearl glue solution and solidify and pellet the solid content to obtain the superfine enzymolysis pearl powder. The prepared pearl powder reserves various amino acids and trace elements and other active components in the pearl. The calcium carbonate in the pearl is converted into active calcium easily absorbed by human body. The keratin insoluble in the gastric acid is converted into easily absorbed free amino acid through proteolysis.

A Chinese invention patent (application No.: 200710141569.X; date of application: August 7, 2007) reveals a method to prepare pearl bioactive peptide. The method is implemented by abstracting the pearl from the fresh and living pearl shells and washed with water. Then the pearl is mixed with the deionized water. The phosphate buffered solution is used to regulate the pH value of the mixed liquor to neutrality-to-acidity. Then the serrapeptass and pearl will undergo hydrolysis reaction. The filter screen is used for filtration and separation to obtain clear liquor and pearl. Then the reaction liquid is boiled in water for enzyme deactivation. Then the solution obtained is spray-dried to obtain pearl bioactive peptide.

A Chinese invention patent (application No.: 200910114409.5; date of application: September 18, 2009) reveals a method to prepare pearl hydrolysate. The steps are as follows: 1) Take some pearl powder and put it into the aqueous hydrochloric acid solution and stir it up to allow for full reaction. Then regulate the pH value of the mixed liquor using ammonium hydroxide to neutrality; centrifuge the mixed liquor to obtain Sediment A; 2) Dissolve Sediment A in purified water and regulate the pH value to 7.5 to 8.5 with ammonium hydroxide; centrifuge the mixed liquor to obtain Sediment B; 3) Dissolve Sediment B in purified water and add enzyme for enzymolysis; centrifuge the enzymatic hydrolysate to obtain the liquid supernatant and Sediment C; the abovementioned enzyme should be the mixture of snail protease and papain. The amount of the enzyme should be 0.3-1.05% of the weight of Sediment B; 4) Dissolve Sediment C in purified water and add enzyme for secondary enzymolysis; centrifuge the mixed liquor and collect the liquid supernatant; the enzyme should be the mixture of trypsin, papain and neutral protease. The amount of the enzyme should be 0.2-0.45% of the weight of Sediment B; 5) Put the liquid supernatants obtained and boil the mixed liquor, which is filtered using nanofiltration membrane with a hole diameter of 1 to 3nm to obtain the product.

### Technical problems

To solve the above technical problems, the purpose of this invention is to provide a method to separate and prepare pearl abstract from pearl. The mechanochemistry principle is adopted for the method, which is implemented by pre-treating pearl powder to improve the reactive surface area and crystal structure of the pearl powder, thus quickly separating the functional components of the pearl in a non-destructive manner and obtain the pearl liquid calcium and pearl compound whitening factor solution. The ultimate purpose is to provide raw materials for pearl skin care products and health care products.

### Technical solutions

To realize the above purposes, this invention adopts the following technical schemes: A method to separate and prepare pearl abstracts from pearl, including the following steps:
1) Smash the pearl powder into nm-level particles and add 5 to 10 portions of deionized water. Apply pressure of 0.5 to 20MPa at a temperature of 30 to 60°C, so as to obtain microemulsion of the pearl powder.
2) Carry out electroosmosis of the pearl powder emulsion at a pressure of 30 to 40V and velocity of 60 to 80L/h. Put deionized water in the thick water tank and pearl powder emulsion in the fresh water tank. The volume ratio of deionized water to pearl powder emulsion is 1:15 to 1:2. Carry out circulated decalcification for 1 to 3 hours. The saline solution containing calcium and pearl protein solution are obtained through separation.
3) The calcium-containing saline solution can be used as the raw material of soluble edible calcium or soluble edible calcium.
4) Add the pearl protein solution obtained into homogeneous phosphate buffer with pH of 6.5 to 7.5 with volume 5 to 20 times of the pearl protein solution.
5) Carry out pre-treatment for 20 to 60 minutes at temperature of 100 to 120°C and pressure of 0.15 to 0.2MPa. Then put the mixed liquor into a reactive pan and add neutral protease with system quality of 0.5% to 2%. Allow it to undergo enzymic catalytic reaction for 4 to 8 hours at temperature of 40 to 70°C. Afterwards, boiling water bath is used as a means of enzyme deactivation for 5 to 15 minutes. Centrifuge the mixed liquor and remove the liquid supernatant to obtain the pearl compound whitening factor solution.

Preferably, the particle size of the selected pearl powder should be 10 to 100nm.

Preferably, the volume ratio of deionized water to pearl powder emulsion should be 1:10 to 1:2.

Preferably, the calcium-containing saline solution should be filtered to obtain transparent clear liquid, which will undergo sterilization and sterile filling, thus finally obtaining pearl soluble liquid calcium.

Preferably, the calcium-containing saline solution obtained should be vacuum dried and ground into powder-like pearl protein health care products and cosmetics raw materials.

Preferably, the abovementioned pearl compound whitening factor solution should undergo sterilization and sterile filling and covering to obtain pearl compound whitening factor solution.

### Beneficial effects

Above technical schemes are adopted for this invention and the functional components are separated from the pearl powder. The mechanochemistry principle is also adopted for preparation. The use of mechanical method allows for separation and abstraction of critical components from the pearl powder without destructing the activity of components of pearl powder. The enzymolysis process after obtaining and purification of protein in the pearl powder allows for the full play of the whitening function of the pearl powder. Application of this method greatly expands the application field of pearl powder and helps refining of the deep-processing application of pearl powder.

### Description of the attached figures

Fig. 1 Method of Embodiment 1: The Curve Graph of Conductivity of Pearl Powder Emulsion Obtained through Sampling and Testing at an Interval of 15 Minutes
Fig. 2 Method of Embodiment 1: The Curve Graph of Calcium Salt Content in Pearl Powder Emulsion Obtained through Sampling and Testing at an Interval of 15 Minutes

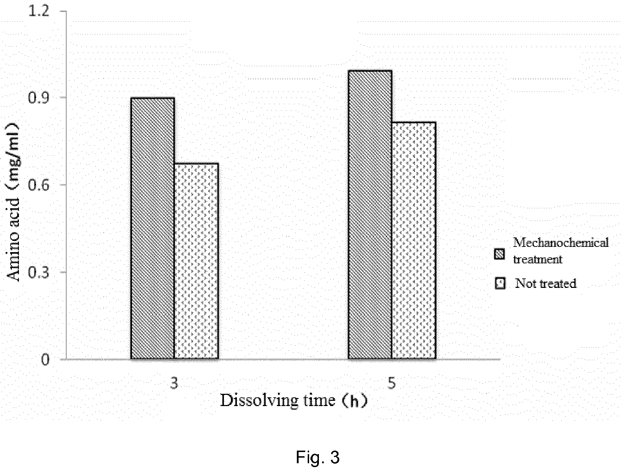

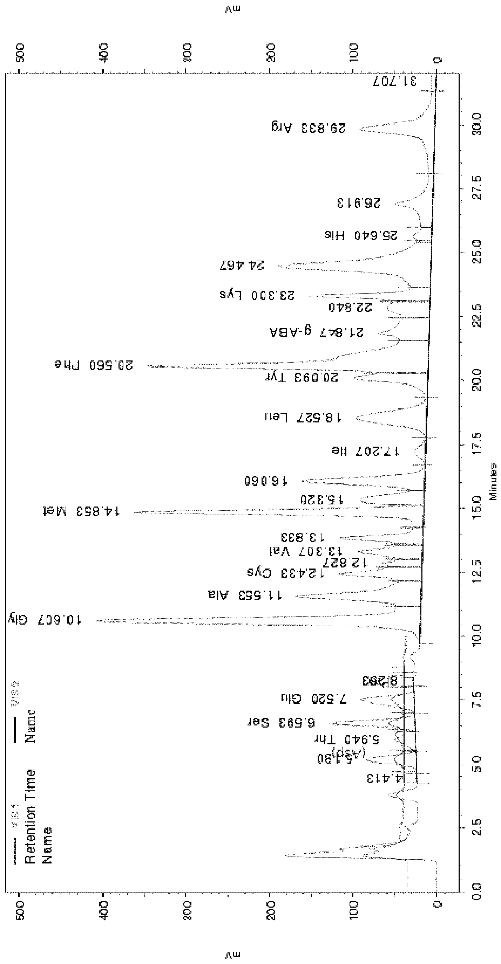

Fig. 3 Method of Embodiment 1: The Obviously Improved Pearl Protein Enzymolysis Effect of Pearl Protein after Pressure and Temperature Treatment as against that before Such Treatment

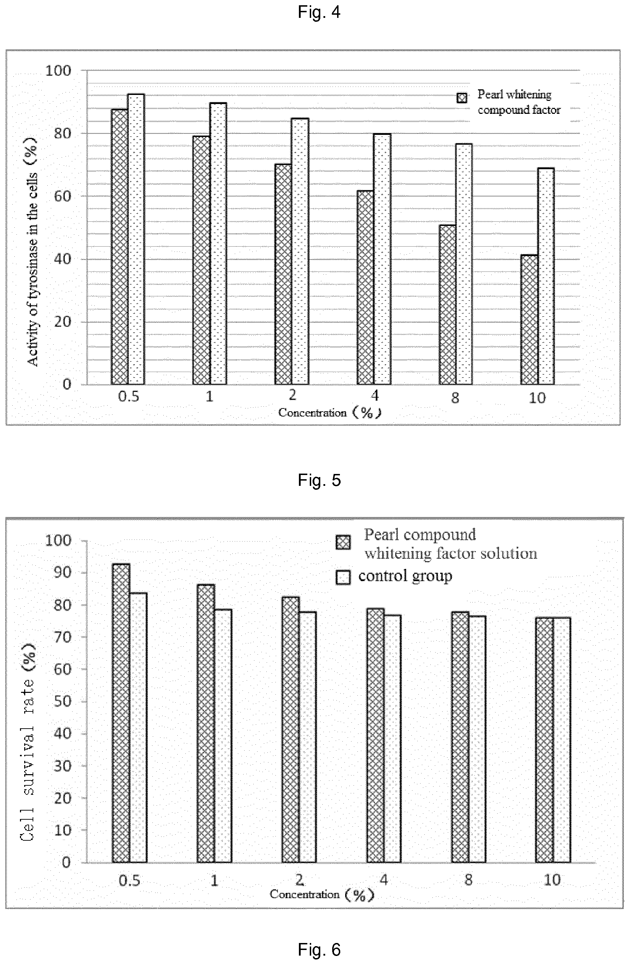

Fig. 4 Analysis Graph of Components of Pearl Compound Whitening Factor Solution Obtained in Embodiment 1
Fig. 5 Graph of Impact of Pearl Compound Whitening Factors Obtained in Embodiment 1 on Activity of Tyrosinase
Fig. 6 Graph of Impact of Pearl Compound Whitening Factor Solution Obtained in Embodiment 1 on Growth of Cells

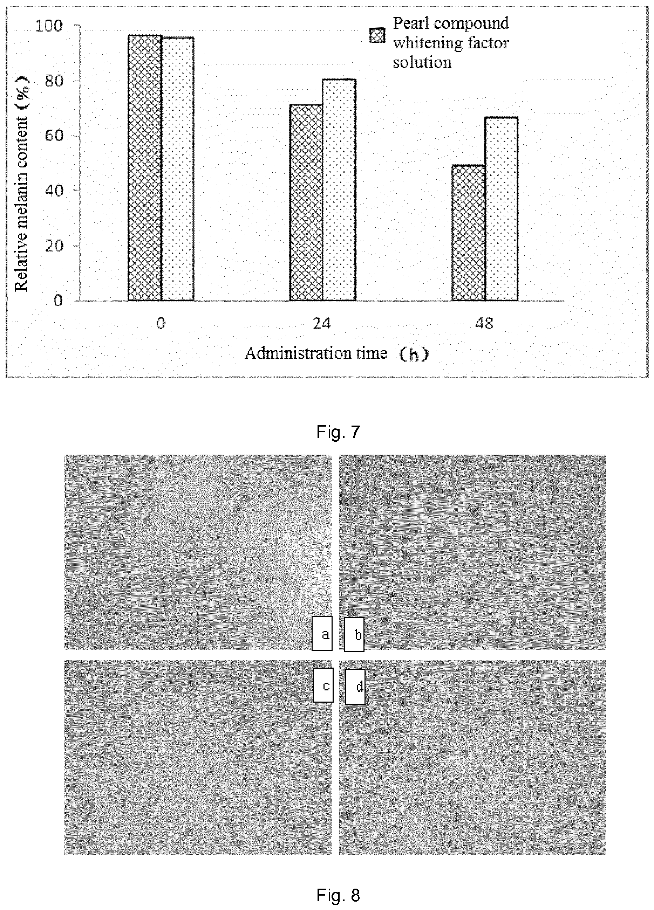

Fig. 7 Graph of Impact of Pearl Compound Whitening Factors Obtained in Embodiment 1 on Content of Melanin in Cells
Fig. 8 Impact of Pearl Compound Whitening Factors on Cells as Observed with a Microscope, a and c respectively represent the growth of cells within 24h and 48h after addition of pearl compound whitening factors.

b and d respectively represent the growth of cells within 24 hours and 48 hours without addition of pearl compound whitening factors.

### Implementation method of this invention

This invention is further explained by embodiments. However, this invention is not limited to the following embodiments.

### Embodiment 1

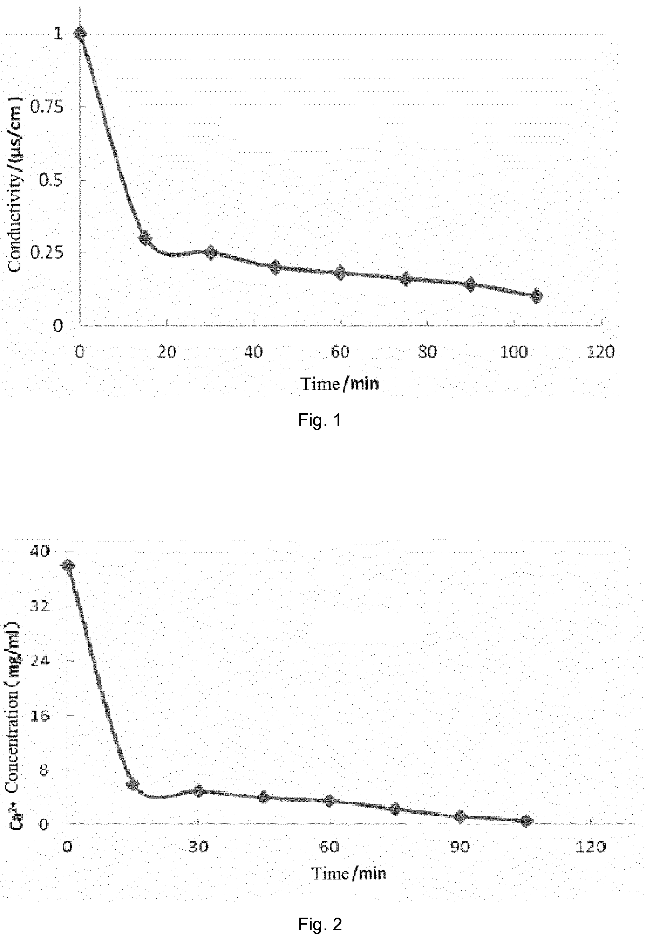

A method to obtain soluble edible calcium from pearl through separation: The following raw materials and steps are involved: Smash the powder into nm-level particles (10 - 100nm) and add 6 portions of deionized water. Apply pressure of 1 MPa at a temperature of 50°C, so as to obtain microemulsion of the pearl powder. Carry out electroosmosis of the pearl powder emulsion at a pressure of 30 to 40V and velocity of 60 to 80L/h. Put deionized water of 5L in the thick water tank and pearl powder emulsion of 15L in the fresh water tank. Carry out circulated decalcification (Ca2+) for 1 to 3 hours. Obtain the content of calcium salt and conductivity of pearl powder emulsion through sampling and testing at an interval of 15 minutes. The testing results are shown in Fig. 1 and Fig. 2. Filter the obtained calcium-containing saline solution to obtain transparent clear liquor, which will undergo sterilization and sterile filling. The product will be pearl soluble liquid calcium.

Add the pearl protein solution obtained into homogeneous phosphate buffer with pH of 7.0 with volume 10 times of the pearl protein solution. Carry out pre-treatment for 40 minutes at temperature of 120°C and pressure of 0.15 MPa. Then put the mixed liquor into a reactive pan and add neutral protease with system quality of 1.0%. Allow it to undergo enzymic catalytic reaction for 6 hours at temperature of 40°C. Afterwards, boiling water bath is used as a means of enzyme deactivation for 5 to 15 minutes. Centrifuge the mixed liquor and remove the liquid supernatant to obtain the pearl compound whitening factor solution.

### Embodiment 2

A method to obtain soluble edible calcium from pearl through separation: The following raw materials and steps are involved: Smash the powder into nm-level particles (10 to 100nm) and add 10 portions of deionized water. Apply pressure of 10MPa at a temperature of 40°C, so as to obtain microemulsion of the pearl powder. Carry out electroosmosis of the pearl powder emulsion at a pressure of 30 to 40V and velocity of 60 to 80L/h. Put deionized water of 5L in the thick water tank and pearl powder emulsion of 10L in the fresh water tank. Carry out circulated decalcification (Ca2+) for 2 hours. Vacuum dry the calcium-containing saline solution and grind the solid content into powder-like pearl protein health care products and cosmetics raw materials.

Add the pearl protein solution obtained into homogeneous phosphate buffer with pH of 6.5 with volume 15 times of the pearl protein solution. Carry out pre-treatment for 40 minutes at temperature of 100°C and pressure of 0.2 MPa. Then put the mixed liquor into a reactive pan and add neutral protease with system quality of 1.0%. Allow it to undergo enzymic catalytic reaction for 6 hours at temperature of 40°C. Afterwards, boiling water bath is used as a means of enzyme deactivation for 5 to 15 minutes. Centrifuge the mixed liquor and remove the liquid supernatant to obtain the pearl compound whitening factor solution.

### Embodiment 3

A method to obtain soluble edible calcium from pearl through separation: The following raw materials and steps are involved: Smash the powder into nm-level particles (10 to 100nm) and add 10 portions of deionized water. Apply pressure of 15MPa at a temperature of 30°C, so as to obtain microemulsion of the pearl powder. Carry out electroosmosis of the pearl powder emulsion at a pressure of 30 to 40V and velocity of 60 to 80L/h. Put deionized water of 2L in the thick water tank and pearl powder emulsion of 10L in the fresh water tank. Carry out circulated decalcification (Ca2+) for 2 hours. Vacuum dry the calcium-containing saline solution and grind the solid content into powder-like pearl protein health care products and cosmetics raw materials.

Add the pearl protein solution obtained into homogeneous phosphate buffer with pH of 7.5 with volume 8 times of the pearl protein solution. Carry out pre-treatment for 60 minutes at temperature of 120°C and pressure of 0.15 MPa. Then put the mixed liquor into a reactive pan and add neutral protease with system quality of 1.0%. Allow it to undergo enzymic catalytic reaction for 5 hours at temperature of 50°C. Afterwards, boiling water bath is used as a means of enzyme deactivation for 5 to 15 minutes. Centrifuge the mixed liquor and remove the liquid supernatant to obtain the pearl compound whitening factor solution.

### Embodiment 1

### 1. Analysis of components of pearl compound whitening factor solution prepared in Embodiment 1

The following data have been obtained through precision measurement using amino acid analyzer, Kjeldahl apparatus and atomic absorption spectrophotometer. The data indicate that compared to the original hydrolyzed pearl liquid protein content, which was > 0.15%, the protein content in the compound whitening factor solution is 1.58%, which is remarkably increased. No calcium ion is detected, indicating that there is no calcium element in the solution.

**Table 1: Analysis Table of Components of Pearl Compound Whitening Factor Solution**

| Name | Content, g/100g | Name | Content, g/100g |
|---|---|---|---|
| Asparaginic acid | 0.7 | Isoleucine | 0.2 |
| Threonine | 0.2 | Leucine | 0.4 |
| Serine | 0.4 | Tyrosine | 0.3 |
| Glutamic acid | 0.4 | Phenylalanine | 0.2 |
| Praline | 0.1 | Lysine | 0.3 |
| Glycine | 0.8 | Histidine | 0.1 |
| Alanine | 0.7 | Arginine | 0.2 |
| Valine | 0.2 | Total | 5.2 |
| Protein content | 1.58 | Calcium ion | Not detected |

### 2. Molecular weight analysis

The liquid phase tandem mass spectrum analysis results indicate that the effective component molecular weight in the pearl compound whitening factor is within 1,000Da.This means that the protein enzymolysis is complete. The polypeptides include oligopeptides, tripeptide, dipeptide and various amino acids. They can be easily absorbed by our skin and help with exercise of activity of the polypeptides and amino acids.

### A. Restraining melanin

### ① Impact of pearl compound whitening factor solution on tyrosinase

The pearl compound whitening factor can significantly restrain the activity of tyrosinase in cells. At a concentration of 8%, the pearl compound whitening factor reaches its half mean inactivation dose for activity of tyrosinase in cells.

### ② Impact of pearl compound whitening factor solution on B16 melanoma cells

### A. Impact on cell growth

According to the growth of B16 cells, there is no impact on survival rate of cells when the sample concentration is 10%. Compared to the control, pearl compound whitening factors facilitate growth of cells.

### B. Impact on melanin in cells

The results showed that the pearl compound whitening factor solution at a concentration of 4% could obviously reduce relative content of melanin.

## Claims

1. A method to obtain soluble edible calcium from pearl through separation, featuring the following steps:
1) Smash the pearl powder into nm-level particles and add 5 to 10 portions of deionized water. Apply pressure of 0.5 to 20MPa at a temperature of 30 to 60°C, so as to obtain microemulsion of the pearl powder;
2) Carry out electroosmosis of the pearl powder emulsion at a pressure of 30 to 40V and velocity of 60 to 80L/h. Put deionized water in the thick water tank and pearl powder emulsion in the fresh water tank. The volume ratio of deionized water to pearl powder emulsion is 1:15 to 1:2. Carry out circulated decalcification for 1 to 3 hours. The saline solution containing calcium and pearl protein solution are obtained through separation;
3) The calcium-containing saline solution can be used as the raw material of soluble edible calcium or soluble edible calcium;
4) Add the pearl protein solution obtained into homogeneous phosphate buffer with pH of 6.5 to 7.5 with volume 5 to 20 times of the pearl protein solution;
5) Carry out pre-treatment for 20 to 60 minutes at temperature of 100 to 120°C and pressure of 0.15 to 0.2MPa. Then put the mixed liquor into a reactive pan and add neutral protease with system quality of 0.5% to 2%. Allow it to undergo enzymic catalytic reaction for 4 to 8 hours at temperature of 40 to 70°C. Afterwards, boiling water bath is used as a means of enzyme deactivation for 5 to 15 minutes. Centrifuge the mixed liquor and remove the liquid supernatant to obtain the pearl compound whitening factor solution.

2. The method described in Claim 1 used to separate and prepare pearl abstract from pearl features a particle size of pearl powder of 10 to 100nm.

3. The method described in Claim 1 used to separate and prepare pearl abstract from pearl features a volume ratio of deionized water to pearl powder emulsion of 1:10 to 1:2.

4. The method described in Claim 1 used to separate and prepare pearl abstract from pearl features filtering the obtained calcium salt solution to obtain transparent clear liquid, which will undergo sterilization and sterile filling to become pearl soluble liquid calcium.

5. The method described in Claim 1 used to separate and prepare pearl abstract from pearl features vacuum-drying the obtained calcium salt solution and grinding the solid content into powder-like pearl protein raw materials of health-care products and cosmetics.

6. The method described in Claim 1 used to separate and prepare pearl abstract from pearl features sterilization and sterile filling of the pearl compound whitening factor solution, which will be covered to make the pearl compound whitening factor solution.

## Patentansprüche

1. Verfahren zur Gewinnung von löslichem genießbarem Calcium aus Perlen durch Trennung, umfassend die folgenden Schritte:
1) Zerkleinern von Perlenpulver in Partikel im nm-Bereich und Zusetzen von 5 bis 10 Teilen deionisiertes Wasser; Anwenden eines Drucks von 0,5 bis 20 MPa bei einer Temperatur von 30 bis 60 °C, um eine Mikroemulsion des Perlenpulvers zu erhalten;
2) Durchführen einer Elektroosmose mit der Perlenpulveremulsion bei einem Druck von 30 bis 40 V und einer Geschwindigkeit von 60 bis 80 L/h; Einfüllen von deionisiertem Wasser in den Trübwasserbehälter und von Perlenpulveremulsion in den Frischwasserbehälter, wobei das Volumenverhältnis von deionisiertem Wasser zu Perlenpulveremulsion 1:15 bis 1:2 beträgt; Durchführen einer Umwälz-Entkalkung während 1 bis 3 Stunden; Gewinnen einer Calcium enthaltenden Salzlösung und einen Perlenproteinlösung durch Trennung;
3) die calciumhaltige Salzlösung kann als Ausgangsmaterial für lösliches genießbares Calcium oder lösliches genießbares Calcium verwendet werden;
4) Zusetzen der gewonnenen Perlenproteinlösung zu einem homogenen Phosphatpuffer mit einem pH von 6,5 bis 7,5 und mit einem Volumen im Ausmaß des 5- bis 20-Fachen der Perlenproteinlösung;
5) Durchführen einer Vorbehandlung während 20 bis 60 Minuten bei einer Temperatur von 100 bis 120 °C und einem Druck von 0,15 bis 0,2 MPa; anschließendes Einfüllen der Mischflüssigkeit in ein Reaktionsgefäß und Zugeben von neutraler Protease mit einer Systemqualität von 0,5 % bis 2 %; Ermöglichen einer enzymatischen katalytischen Reaktion während 4 bis 8 Stunden bei einer Temperatur von 40 bis 70 °C, anschließendes Verwenden eines kochenden Wasserbads während 5 bis 15 Minuten als Mittel zur Enzymdeaktivierung; Zentrifugieren der Mischflüssigkeit und Entfernen des flüssigen Überstands, um die Perlenverbindungs-Bleichfaktorlösung zu gewinnen.

2. Verfahren nach Anspruch 1, das dazu verwendet wird, einen Perlenauszug aus Perlen abzutrennen und herzustellen, wobei die Partikelgröße des Perlenpulvers 10 bis 100 nm beträgt.

3. Verfahren nach Anspruch 1, das dazu verwendet wird, einen Perlenauszug aus Perlen abzutrennen und herzustellen, wobei ein Volumenverhältnis von deionisiertem Wasser zu Perlenpulveremulsion 1:10 bis 1:2 beträgt.

4. Verfahren nach Anspruch 1, das dazu verwendet wird, einen Perlenauszug aus Perlen abzutrennen und herzustellen, wobei die gewonnene Calciumsalzlösung filtriert wird, um eine durchsichtige klare Flüssigkeit zu gewinnen, die einer Sterilisation und sterilen Abfüllung unterzogen wird, um lösliches flüssiges Calcium aus Perlen zu ergeben.

5. Verfahren nach Anspruch 1, das dazu verwendet wird, einen Perlenauszug aus Perlen abzutrennen und herzustellen, wobei die gewonnene Calciumsalzlösung vakuumgetrocknet wird und der Feststoffgehalt zu pulverförmigen Perlenprotein-Ausgangsmaterialien für Gesundheitsprodukte und Kosmetika gemahlen wird.

6. Verfahren nach Anspruch 1, das dazu verwendet wird, einen Perlenauszug aus Perlen abzutrennen und herzustellen, umfassend eine Sterilisation und sterile Abfüllung der Perlenverbindungs-Bleichfaktorlösung, die abgedeckt wird, um die Perlenverbindungs-Bleichfaktorlösung herzustellen.

## Revendications

1. Procédé d'obtention de calcium comestible soluble à partir de perles par séparation, présentant les étapes suivantes consistant à :
1) Briser la poudre de perles en des particules de niveau nanométrique et ajouter entre 5 et 10 parties d'eau désionisée. Appliquer une pression comprise entre 0,5 et 20 MP à une température comprise entre 30 et 60 °C, de façon à obtenir une microémulsion de la poudre de perles ;
2) Effectuer une électro-osmose de l'émulsion de poudre de perles à une pression comprise entre 30 et 40 V et à une vélocité comprise entre 60 et 80 L/h. Insérer l'eau désionisée dans le réservoir d'eau épaissie et l'émulsion de poudre de perles dans le réservoir d'eau fraîche. Le rapport volumique d'eau désionisée à l'émulsion de poudre perle est compris entre 1:15 et 1:2. Effectuer la décalcification circulée pendant 1 à 3 heures. La solution saline contenant le calcium et la solution protéique de perles sont obtenues par séparation ;
3) La solution saline contenant le calcium peut être utilisée en tant que matière première de calcium comestible soluble ou le calcium comestible soluble ;
4) Ajouter la solution protéique de perles obtenue à l'intérieur d'un tampon de phosphate homogène de pH compris entre 6,5 et 7,5 avec un volume compris entre 5 et 20 fois le volume de la solution protéique de perles ;
5) Effectuer un prétraitement pendant 20 à 60 minutes à une température comprise entre 100 et 120 °C et à une pression comprise entre 0,15 et 0,2 MPa. Placer ensuite la liqueur mixte à l'intérieur d'une nacelle réactive et ajouter une protéase neutre possédant une qualité du système comprise entre 0,5 % et 2 %. Lui permettre d'effectuer une réaction catalytique enzymatique pendant 4 à 8 heures à une température comprise entre 40 et 70 °C. Ensuite, un bain-marie bouillon est utilisé comme moyen de désactivation d'enzymes pendant 5 à 15 minutes. Centrifuger la liqueur mixte et éliminer le surnageant liquide pour obtenir la solution de facteur de blanchissement de composé de perles.

2. Procédé selon la revendication 1 utilisé pour séparer et préparer un extrait de perles à partir de perles présentant une taille particulaire de poudre de perles comprise entre 10 et 100 nm.

3. Procédé selon la revendication 1 utilisé pour séparer et préparer un extrait de perles à partir de perles présentant un rapport volumique d'eau désionisée à une émulsion de poudre de perles compris entre 1:10 et 1:2.

4. Procédé selon la revendication 1 utilisé pour séparer et préparer un extrait de perles à partir de perles présentant une filtration de la solution de sel de calcium obtenue pour obtenir un liquide clair transparent, qui subira une stérilisation et un remplissage stérile pour devenir une perle de calcium liquide soluble.

5. Procédé selon la revendication 1 utilisé pour séparer et préparer un extrait de perles à partir de perles présentant un séchage sous vide de la solution de sel de calcium obtenue et un broyage des matières solides dans les matières premières protéiques de perles de type poudre des produits de beauté et cosmétiques.

6. Procédé selon la revendication 1 utilisé pour séparer et préparer un extrait de perles à partir de perles présentant des caractéristiques de stérilisation et un remplissage stérile de la solution de facteur de blanchiment de composé de perles, qui sera recouvert pour préparer la solution de facteur de blanchiment de composé de perles.
